Europäisches Patentamt

European Patent Office

Office européen des brevets

(11) Veröffentlichungsnummer: **0 256 402**
A2

## (12) EUROPÄISCHE PATENTANMELDUNG

(21) Anmeldenummer: **87111145.6**

(22) Anmeldetag: **01.08.87**

(51) Int. Cl.⁴: **A61K 45/06** , **A61K 37/64** , **A61K 31/54** , **A61K 31/44** , **A61K 31/40** , //(A61K37/64,31:44),(A61K31/5-4,31:44),(A61K31/44,31:40)

(30) Priorität: **14.08.86 DE 3627613**

(43) Veröffentlichungstag der Anmeldung:
**24.02.88 Patentblatt 88/08**

(84) Benannte Vertragsstaaten:
**AT BE CH DE ES FR GB LI NL**

(71) Anmelder: **Merck Patent Gesellschaft mit beschränkter Haftung**
**Frankfurter Strasse 250**
**D-6100 Darmstadt(DE)**

(72) Erfinder: **Jonas, Rochus, Dr.**
**Kesselhutweg 12**
**D-6100 Darmstadt(DE)**
Erfinder: **Sombroek, Johannes, Dr.**
**Robert-Koch-Strasse 32**
**D-6100 Darmstadt(DE)**
Erfinder: **Klockow, Michael, Dr.**
**Fuchsenhütte 42**
**D-6101 Rossdorf 1(DE)**

(54) **Pharmazeutische Zubereitung.**

(57) Die ErFindung betrifft eine neue pharmazeutishe Zubereitung, enthaltend mindestens ein positiv-inotropes Imidazopyridin und/oder mindestens eines seiner physiologisch unbedenklichen Salze und mindestens einen ACE-Hemmer sowie die Verwendung dieser Zubereitung bei der Bekämpfung der Herzinsuffizienz.

EP 0 256 402 A2

## Pharmazeutische Zubereitung

Die Erfindung betrifft eine neue pharmazeutische Zubereitung, enthaltend mindestens ein positiv-inotropes Imidazopyridin und/oder mindestens eines seiner physiologisch unbedenklichen Salze und mindestens einen ACE-Hemmer sowie die Verwendung dieser Zubereitung bei der Bekämpfung der Herzinsuffizienz.

Unter den Imidazopyridinen sind Imidazo[4,5-c]pyridine, insbesondere 2-Aryl-imidazo[4,5-c]-pyridine (vgl. DE-OS 3139064) bevorzugt, ferner Imidazo[4,5-b]pyridine, insbesondere 2-Aryl-imidazo[4,5-b]pyridine (vgl. DE-OS 2305339, DE-OS 3132754). Besonders bevorzugt sind Isomazol [2-(2-Methoxy-4-sulfinylmethylphenyl)-imidazo[4,5-c]pyridin] und dessen physiologisch unbedenkliche Salze, bevorzugt das Hydrochlorid und das Fumarat.

ACE (= angiotensin converting enzyme)-Hemmer sind Substanzen, die die Wirkung von Enzymen hemmen, welche Angiotensin I in Angiotensin II umwandeln. ACE-Hemmer senken den Blutdruck, wenn der Bluthochdruck auf Angiotensin II zurückgeführt werden kann. Als Vasodilatatoren weisen sie ein günstiges hämodynamisches Profil auf.

Bevorzugte ACE-Hemmer sowie Verfahren zu ihrer Herstellung sind z. B. in der DE-OS 3436386, der DE-OS 3437917 und der EP-OS 12401 angegeben. Es seien z. B. diejenigen folgender Formel genannt:

$$R\text{-}CO\text{-}CR^1R^2\text{-}NH\text{-}CHR^3\text{-}CO\text{-}NR^4\text{-}CR^5R^7\text{-}CO\text{-}R^6$$

worin

R und $R^6$ gleich oder verschieden sind und Hydroxy, Niedrigalkoxy, Niedrigalkenoxy, Di-niedrigalkylamino-niedrigalkoxy, Acylamino-niedrigalkoxy, Acyloxy-niedrigalkoxy, Aryloxy, Ar-niedrigalkoxy, substituiertes Aryloxy oder substituiertes Ar-niedrigalkoxy, worin der Substituent Methyl, Halogen oder Methoxy ist, Amino, Niedrigalkylamino, Di-niedrigalkylamino, Aryl-niedrigalkylamino oder Hydroxyamino sind,

$R^1$ Wasserstoff, Alkyl mit 1 bis 20 Kohlenstoffatomen, das verzweigte,cyclische und ungesättigte Alkylgruppen umfaßt, substituiertes Niedrigalkyl, worin der Substituent Halogen, Hydroxy, Niedrigalkoxy, Aryloxy, Amino, Niedrigalkylamino, Di-niedrigalkylamino, Acylamino, Arylamino, Guanidino, Imidazolyl, Indolyl, Mercapto, Niedrigalkylthio, Arylthio, Carboxy, Carboxamido, Carbo-niedrigalkoxy ist, Phenyl, substituiertes Phenyl, worin der Substituent Niedrigalkyl, Niedrigalkoxy oder Halogen ist, Ar-niedrigalkyl oder Heteroar-niedrigalkyl, Ar-niedrigalkenyl oder Heteroar-niedrigalkenyl, substituiertes Ar-niedrigalkyl, substituiertes Heteroar-niedrigalkyl, substituiertes Ar-niedrigalkenyl oder substituiertes Heteroar-niedrigalkenyl, worin der Substituent Halogen oder Dihalogen, Niedrigalkyl, Hydroxy, Niedrigalkoxy, Amino, Aminomethyl, Acylamino, Di-niedrigalkylamino, Niedrigalkylamino, Carboxyl, Halogen-niedrigalkyl, Cyano oder Sulfonamido ist; Ar-niedrigalkyl oder Heteroar-niedrigalkyl, das am Alkylteil durch Amino oder Acylamino substituiert ist, bedeutet,

$R^2$ und $R^7$ Wasserstoff oder Niedrigalkyl bedeuten,

$R^3$ Wasserstoff, Niedrigalkyl, Phenyl-niedrigalkyl, Aminomethylphenyl-niedrigalkyl, Hydroxyphenylniedrigalkyl, Hydroxy-niedrigalkyl, Acylaminoniedrigalkyl, Amino-niedrigalkyl, Dimethylaminoniedrigalkyl, Halogen-niedrigalkyl, Guanidino-niedrigalkyl, Imidazolyl-niedrigalkyl, Indolyl-niedrigalkyl, Mercaptoniedrigalkyl und Niedrigalkylthio-niedrigalkyl ist,

$R^4$ Wasserstoff oder Niedrigalkyl ist,

$R^5$ Wasserstoff, Niedrigalkyl, Phenyl, Phenyl-niedrigalkyl, Hydroxyphenyl-niedrigalkyl, Hydroxy-niedrigalkyl, Amino-niedrigalkyl, Guanidino-niedrigalkyl, Imidazolyl-niedrigalkyl, Indolylniedrigalkyl, Mercaptoniedrigalkyl oder Niedrigalkylthio-niedrigalkyl ist,

$R^4$ und $R^5$ miteinander unter Bildung einer Alkylenbrücke mit 2 bis 4 Kohlenstoffatomen, einer Alkylenbrücke mit 2 bis 3 Kohlenstoffatomen und einem Schwefelatom, einer Alkylenbrücke mit 3 bis 4 Kohlenstoffatomen, die eine Doppelbindung oder eine Alkylenbrücke wie oben enthält, substituiert durch Hydroxy, Niedrigalkoxy, Niedrigalkyl oder Di-niedrigalkyl, verbunden sein können,

und die pharmazeutisch annehmbaren Salze davon.

Bevorzugte ACE-Hemmer im einzelnen sind Captopril (SQ-14225), Enalapril (MK-421), Lisinopril (MK-521) und Pivopril (Pivalopril, RHC-3659), weiterhin Alacepril (DU-1219), Ancovenin, Cilazapril (Ro-31-2848), Cilazaprilat, Delapril (Rev 6000A), Enalaprilat (MK-422), Foroxymithine, Fosenopril (SQ-28555), Indalapril (REV-6000A), Indolapril (CI-907), Nicotianamin, Pentopril (CGS-13945), Perindopril (S-9490-3), Phenacein, Pivopril, Quinapril (CI-906), Ramipril (Hoe-498), Rentiapril (SA-446), Spirapril, Teprotid, Trandolapril, Zofenopril (SQ-26991), 15-B-2, BRL-36378, CGS-13928-C, CGS-14824-A, CGS-16617, CI-908, CI-925, CL-242817, CV-3317, EU 5476, K-26, L-681176, MC-838, MS-41, Sch-33844, SQ 28555, SQ-26900, Wy-44221, Y-108, YS-980 (SA-291).

Gleichzeitige Gabe von ACE-Hemmern verstärkt bemerkenswerterweise deutlich die positiv-inotrope Wirkung der genannten Imidazopyridine; gleichzeitig wird der pathologisch erhöhte periphere Widerstand gesenkt. Diese Effekte können z. B. in Standardtests an narkotisierten oder wachen Hunden, Katzen, Affen oder Minischweinen, die positiv-inotropen Wirkungen auch an isolierten Herzpräparationen (z. B. Vorhof, Papillarmuskel oder perfundiertes Ganzherz) von Ratte, Meerschweinschen oder Katze ermittelt werden, z. B. nach Methoden, wie sie beschrieben sind in Arzneimittelforschung, Band 31 (I), Nr. 1a (1981), Seiten 141 bis 170; US-PS 4584299.

Gemische bestimmter ACE-Hemmer mit bestimmten Dihydropyridazinonen sind aus der US-PS 4584299 bekannt. Die letztgenannten wirken ebenfalls positiv-inotrop; jedoch besitzen sie eine chemische Konstitution, die mit der der vorliegenden Imidazopyridine nicht vergleichbar ist.

Die neue pharmazeutische Zubereitung kann hergestellt werden, indem man mindestens ein positiv-inotrop wirksames Imidazopyridin, vorzugsweise Isomazol, und/oder mindestens eines seiner physiologisch unbedenklichen Salze zusammen mit mindestens einem ACE-Hemmer sowie zusammen mit mindestens einem Träger-oder Hilfsstoff in eine geeignete Dosierungsform bringt. Die so erhaltenen Zubereitungen können als Arzneimittel in der Human-oder Veterinärmedizin eingesetzt werden. Als Trägersubstanzen kommen organische oder anorganische Stoffe in Frage, die sich für die enterale (z. B. orale oder rektale), parenterale oder topische Applikation eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen, zur rektalen Anwendung Suppositorien, zur parenteralen Applikation Lösungen, vorzugsweise ölige oder wässerige Lösungen, ferner Suspensionen, Emulsionen oder Implantate, für die topische Anwendung Salben, Cremes oder Pflaster. Die Wirkstoffe können auch lyophilisiert und die erhaltenen Lyophilisate z. B. zur Herstellung von Injektionspräparaten verwendet werden. Die Zubereitungen können sterilisiert sein und/oder Hilfsstoffe wie Konservierungs-, Stabilisierungs-und/oder Netzmittel, Emulgatoren, Salze zur Beeinflussung des osmotischen Druckes, Puffersubstanzen, Farb-und/oder Aromastoffe enthalten.

Die erfindungsgemäßen Zubereitungen werden in der Regel in Analogie zu bekannten positiv inotrop wirksamen Substanzen, insbesondere Amrinon, Sulmazol oder Isomazol, verabreicht. Die Dosierungen der Imidazopyridine bzw. ihrer Salze liegen vorzugsweise zwischen etwa 10 mg und 500 mg, insbesondere 25 und 250 mg, ganz besonders bevorzugt zwischen 50 und 150 mg pro Dosierungseinheit. Die ACE-Hemmer werden vorzugsweise in Dosierungen zwischen etwa 2 und etwa 500 mg, insbesondere zwischen 5 und 200 mg pro Dosierungseinheit verwendet. Bevorzugte Dosierungen von Captopril liegen zwischen 5 und 200, insbesondere zwischen 25 und 100 mg, von Enalapril zwischen 2 und 30, insbesondere zwischen 10 und 20 mg pro Dosierungseinheit. Die tägliche Dosierung der Imidazopyridine bzw. ihrer Salze liegt vorzugsweise zwischen etwa 0,2 und 10 mg/kg Körpergewicht, diejenige der ACE-Hemmer vorzugsweise zwischen etwa 0,04 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Beispiel 1: Tabletten

Ein Gemisch von 1 kg Isomazol-fumarat, 0,5 kg Captopril, 4 kg Lactose, 1,2 kg Kartoffelstärke, 0,2 kg Talk und 0,1 kg Magnesiumstearat wird in üblicher Weise zu Tabletten gepreßt, derart, daß jede Tablette 50 mg Isomazolfumarat und 25 mg Captopril enthält.

Beispiel 2: Dragees

Analog Beispiel A werden Tabletten gepreßt, die anschließend in üblicher Weise mit einem Überzug aus Saccharose, Kartoffelstärke, Talk, Tragant und Farbstoff überzogen werden.

Beispiel C: Kapseln

Man füllt ein gesiebtes Gemisch von 5 kg Isomazol-hydrochlorid, 1 kg Enalapril-hydrogenmaleat und 6 kg Lactose in üblicher Weise in Hartgelatinekapseln, so daß jede Kapsel 50 mg Isomazol-hydrochlorid und 10 mg Enalaprilhydrogenmaleat enthält.

Beispiel D: Ampullen

Eine Lösung von 1 kg Isomazol-hydrochlorid und 0,1 kg Dinatriumsalz der 1-[N-(1-Carboxy-3-phenylpropyl)alanyl]-thiomorpholin-3-carbonsäure (S,S,S-Form) in 30 1 zweifach destilliertem Wasser wird steril filtriert, in Ampullen abgefüllt, unter sterilen Bedingungen lyophilisiert und steril verschlossen. Jede Ampulle enthält 50 mg Isomazol-hydrochlorid und 5 mg des Dinatriumsalzes der Thiomorpholincarbonsäure.

**Ansprüche**

1. Pharmazeutische Zubereitung, enthaltend mindestens ein positiv-inotrop wirksames Imidazopyridin und/oder mindestens eines seiner physiologisch unbedenklichen Salze und mindestens einen ACE-Hemmer.

2. Pharmazeutische Zubereitung, enthaltend Isomazol und/oder eines seiner physiologisch unbedenklichen Salze und mindestens einen ACE-Hemmer.

3. Verfahren zur Herstellung einer pharmazeutischen Zubereitung, dadurch gekennzeichnet, daß man mindestens ein positiv-inotrop wirksames Imidazopyridin und/oder mindestens eines seiner physiologisch unbedenklichen Salze zusammen mit mindestens einem ACE-Hemmer sowie zusammen mit mindestens einem Träger-oder Hilfsstoff in eine geeignete Dosierungsform bringt.

4. Verwendung einer pharmazeutischen Zubereitung enthaltend mindestens ein positiv-inotrop wirksames Imidazopyridin und/oder mindestens eines seiner physiologisch unbedenklichen Salze und mindestens einen ACE-Hemmer bei der Bekämpfung der Herzinsuffizienz.